# EUROPEAN PATENT APPLICATION

(11) **EP 3 995 570 A1**
(43) Date of publication of application: **11.05.2022**
(21) Application number: 20835482.9
(22) Date of filing: 21.01.2020
(51) Int. Cl.: C12N 5/0735, C12N 5/074

(54) **COMPOSITION FOR PROMOTING PROLIFERATION OF STEM CELLS, CONTAINING, AS ACTIVE INGREDIENT, CP1P OR PHARMACEUTICALLY ACCEPTABLE SALT THEREOF**

(30) Priority: 02.07.2019 KR 20190079547
(71) Applicant: Industry-University Cooperation Foundation Hanyang University, Seoul 04763 (KR); Axceso Biopharma Co., Ltd., Anyang-si, Gyeonggi-do 14056 (KR)
(72) Inventor: KIM, Kye Seong, Seoul 06217 (KR); LIM, Jung Jin, Guri-si Gyeonggi-do 11902 (KR); KIM, Hyung Joon, Seoul 05667 (KR); CHOI, Myeong Jun, Seoul 06286 (KR); KIM, Su Jin, Bucheon-si Gyeonggi-do 14482 (KR)
(74) Representative: dompatent von Kreisler Selting Werner - Partnerschaft von Patent- und Rechtsanwälten mbB
(86) International application number: PCT/KR2020/001037
(87) International publication number: WO 2021/002554

(57) **Abstract**

The present invention relates to: a composition for promoting the proliferation of pluripotent stem cells, containing, as an active ingredient, cP1P or a pharmaceutically acceptable salt thereof; and a composition added to a stem cell culture liquid. When culturing stem cells by using a composition for promoting the proliferation of stem cells and a composition added to a stem cell culture medium, according to the present invention, sternness can be strengthened, growth can be promoted, and apoptosis can be inhibited.

## Description

### [Technical Field]

The present invention relates to a composition for promoting proliferation and enhancing function, and composition for addition to a stem cell culture medium, including cP1P or a pharmaceutically acceptable salt thereof as an active ingredient.

### [Background Art]

Pluripotent stem cells (PSCs) not only may proliferate indefinitely but also have the characteristic of being able to differentiate into all types of cells and tissues constituting the human body so that the cells can be used for the production of disease models in culture dishes, treatment of intractable diseases by inducing differentiation into functional cells, and the like.

To date, PSCs are known to have established more than 1,000 types of human embryonic stem cells (hESCs) and 1,200 types of induced pluripotent stem cells (iPSCs) worldwide. However, most of the stem cell-based research, such as establishment, maintenance culture, storage, and induction of differentiation into specific cells, is conducted in a laboratory environment using a culture medium containing animal-derived sources. The standard for the intrinsic naive state of PSCs also depends on various laboratories, so the standard is ambiguous.

Countries around the world have been trying to secure xeno-independent and GMP-grade stem cells in order to convert established research-grade PSCs into clinical grade. In foreign countries, preliminary work is already underway to develop clinical-grade pluripotent cell therapy, and various clinical research-stage culture media have been developed and marketed. However, although xeno-independent and GMP-grade serum-free culture media are being developed in countries around the world, most of the research is focused on a culture medium for the production of biological products such as antibodies. Currently available clinical research-stage culture medium has a lower cell proliferation effect than serum culture medium. Thus, there are few additives that can completely replace serum. In addition, there is no currently available culture medium for culturing PSCs as a clinical-grade until now because the currently available clinical research-stage culture medium contains very expensive growth factors for use in the industry and thus has a very high production cost.

Recently, the development of a clinically applicable serum-free culture medium for stem cells has been actively conducted mainly in the United States and Japan. In Korea, a number of companies and research institutes have been developing culture media for PSCs culture, but none have succeeded in making a prototype or commercializing it. Thus, they rely 100% on imports. The price of imported serum-free culture medium for research stem cell culture is 400,000 won to 1.5 million won based on 500 mL, which is very expensive. In the case of human PSCs, the difficulty in maintaining and proliferating stem cells in a naive state is increasing because a defined culture system of serum-free and feeder-free is oriented. So far, human PSCs have been cultured and maintained by adding various types of growth factors and inhibitors to the 2i (GSK3b and MEK inhibitors) system in the mouse model, but PSCs in a completely naive state have not been established.

### [Disclosure]

### [Technical Problem]

An object of the present invention is to provide a composition for promoting stem cell proliferation including o-cyclic phytosphingosine-1-phosphate (cP1P) or a pharmaceutically acceptable salt thereof as an active ingredient.

Another object of the present invention is to provide a composition for addition to a stem cell culture medium including cP1P or a pharmaceutically acceptable salt thereof as an active ingredient.

Still another object of the present invention is to provide a method culturing stem cells, including a step of culturing by treating the composition for promoting stem cell proliferation in stem cells.

Yet another object of the present invention is to provide a kit for inhibiting stem apoptosis in an *in vitro* environment including the composition for promoting stem cell proliferation.

### [Technical Solution]

In order to achieve the above objects, the present invention provides a composition for promoting stem cell proliferation including cP1P or a pharmaceutically acceptable salt thereof as an active ingredient.

Further, the present invention provides a composition for addition to a stem cell culture medium including cP1P or a pharmaceutically acceptable salt thereof as an active ingredient.

Further, the present invention provides a method for culturing stem cells, including a step of culturing by treating the composition for promoting stem cell proliferation in stem cells.

Further, the present invention provides a kit for inhibiting stem apoptosis in an *in vitro* environment including the composition for promoting stem cell proliferation.

Further, the present invention provides a method for promoting stem cell proliferation, including a step of culturing stem cells by adding o-cyclic phytosphingosine-1-phosphate (cP1P) or a pharmaceutically acceptable salt thereof to a stem cell culture medium.

### [Advantageous Effects]

The present invention relates to a composition for promoting the proliferation of pluripotent stem cells, containing, as an active ingredient, cP1P or a pharmaceutically acceptable salt thereof; and a composition for addition o a stem cell culture medium. When culturing stem cells by using a composition for promoting the proliferation of stem cells and a composition for addition to a stem cell culture medium, according to the present invention, sternness may be strengthened, proliferation may be promoted, and apoptosis may be inhibited.

### [Description of Drawings]

FIG. 1 shows the chemical structures of o-cyclic phytosphingosine-1-phosphate (cPIP), sphingosine-1-phosphate (SIP), and phytosphingosine-1-phosphate (PIP).
FIG. 2 shows a microscopic view of changes in cell proliferation ability after treating hPSCs with cP1P, PIP, or SIP.
FIG. 3 shows a result of measuring the proliferation rate of cells after treating hPSCs with a concentration of 1 to 500 nM cP1P, PIP, or SIP.
FIG. 4 shows a result of observing hPSCs stained using an alkaline phosphatase staining kit with an optical microscope after treating hPSCs with a control group (vehicle, DMSO) or cP1P.
FIG. 5 shows a result confirming the change in the number and size of colonies after treating hPSCs with a control group (vehicle, DMSO) or cP1P.
FIG. 6 shows a result confirming the change in the total number of cells according to subculture after treating hPSCs with a control group (vehicle, DMSO) or cP1P.
FIG. 7 shows a result confirming the change in cell cycle after treating hPSCs with a control group (vehicle, DMSO) or cP1P.
FIG. 8 shows a result confirming the change in apoptosis after treating hPSCs with a control group (vehicle, DMSO) or cP1P.
FIG. 9 shows a result confirming the change in the pluripotency after treating hPSCs with a control group (vehicle, DMSO) or cP1P.
FIG. 10 shows a result confirming the change in gene expression after long-term treatment of hPSCs with a control group (vehicle, DMSO) or cP1P.
FIG. 11 shows a schematic diagram that stem cells with enhanced pluripotent properties can be mass-produced by treating hPSCs with a culture additive or culture medium containing cP1P.

### [Best Mode]

Hereinafter, the present invention will be described in detail by reference examples and embodiments. However, the following reference examples and embodiments are merely illustrative of the present invention and should not be construed as limiting the present invention.

### Example 1. Confirmation of hPSC cell proliferation ability according to cP1P concentration

In order to confirm the change in the proliferation ability of hPSCs according to the o-cyclic phytosphingosine-1-phosphate (cP1P) concentration, hPSCs were cultured, and cP1P was treated for each concentration, and then the change in the proliferation of hPSCs was confirmed.

Before culturing hPSCs, a culture dish was coated with 10 µg/ml vitronectin XF (STEMCELL) at room temperature for 2 hours, then the coating solution was removed. Essential 8 culture medium (E8, STEMCELL) containing 10 uM Y27632 (ROCK inhibitor, TOCRIS) was added to improve the cell adhesion ability of the culture dish. hPSCs (CHA-ESC15; CHA University of Science and Technology) were inoculated at a density of 2,000 cells/cm² in a culture dish. They were shaken up and down, left and right to evenly disperse the cells and cultured within 24 hours in a 37°C CO₂ incubator. The culture medium was replaced with the E8 culture medium from which Y27632 was removed, and then the cells were cultured by replacing the culture medium every day until the next passage.

While the culture medium was replaced and treated by 50 nM, 100 nM, or 500 nM of cP1P (Korea Patent Publication No. 10-2017-0129460, manufactured by Axceso Bio), SIP (Sigma-Aldrich US, 73914), or PIP (Axceso Bio) in hPSCs subcultured as described above every 2 days, the cells were cultured for 6 days. On the 7th day, the cells were observed using an optical microscope at a magnification of 100 times.

In addition, in order to measure the number of cells, the culture medium of the cells was removed on the 7th day of culture, and they were washed once with PBS. Then, for PBS-removed cells, cell dissociation buffer (STEMCELL) was used to cause single-cell. The separated cells were centrifuged at 1000 rpm for 2 minutes to remove the buffer. Then, the cells were suspended in an E8 medium, and the number of cells was measured using EVE^{™} Automated Cell Counter (NanoEnTek).

Meanwhile, the control group was tested in the same manner as above, except that hPSCs were treated with DMSO (AppliChem GE, A3672,0100).

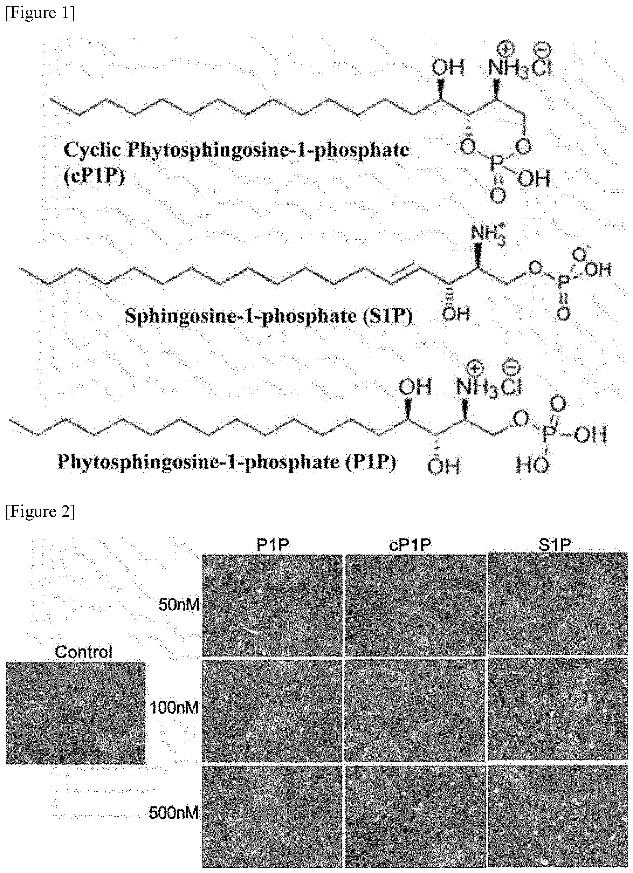

As a result, as shown in FIG. 2, it was confirmed that the cell proliferation ability was significantly increased when hPSCs were treated with cP1P compared to those treated with PIP or SIP. Further, it was confirmed by micrographs that apoptosis occurred when SIP or PIP was treated as a positive control.

### Example 2. Confirmation of hPSCs proliferation ability according to cP1P treatment

In order to determine the optimal concentration of cP1P representing hPSCs proliferation ability, the cell proliferation ability was confirmed at a concentration of 1 nM to 500 nM.

The experiment was performed in the same manner as in Example 1, except that the culture medium was replaced and treated by 1 nM, 10 nM, 50 nM, 100 nM, or 500 nM of PIP, cP1P, or SIP every 2 days in the subcultured hPSC cells or the culture medium was replaced by the control group (vehicle, DMSO) every 2 days. The culture was performed for 6 days, and hPSCs were observed on the 7th day. The proliferation rate was calculated as {(total number of cells in PIP, cP1P, or SIP treatment group)/(total number of cells in the control group)} × 100.

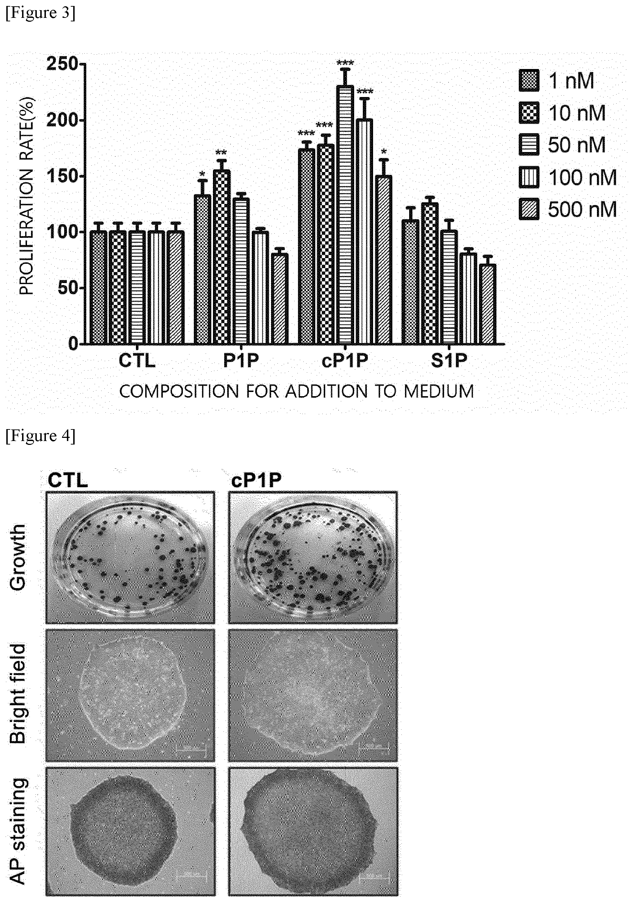

As a result, as shown in FIG. 3, when treated with 10 nM of SIP, the cell proliferation rate was increased by about 1.25 times compared to the control group, and when treated with 10 nM of PIP, the cell proliferation rate was only increased about 1.55 times compared to the control group, but when treated with 10 nM of cP1P, the cell proliferation rate was increased by 1.78 times compared to the control group.

Therefore, it was found that even at a low concentration of 10 nM, the cell proliferation rate was significantly increased when hPSC cells were treated with cP1P compared to the drug used as a positive control.

### Example 3. Confirmation of change in differentiation ability by cP1P treatment

In order to check whether the differentiation ability of hPSCs was changed by cP1P treatment, hPSCs were treated with 10 nM cP1P or a control group (vehicle, DMSO) in the same manner as in Example 2. Then alkaline phosphatase staining kit (Sigma-Aldrich US, SCR004) was used for staining, and the cells were observed with an optical microscope.

As a result, as shown in FIG. 4, it was confirmed that when hPSCs were treated with cP1P, the undifferentiation ability was well maintained compared to the control group.

### Example 4. Confirmation of change in colony number and size by cP1P treatment

Changes in colony number and size by cP1P treatment were confirmed in the following manner.

Specifically, in the same manner as in Example 1, hPSCs were treated with 1 nM, 10 nM, 100 nM, 1,000 nM, or 10,000 nM of cP1P and cultured for 6 days. On the 7th day, the colony number and size were observed with an optical microscope.

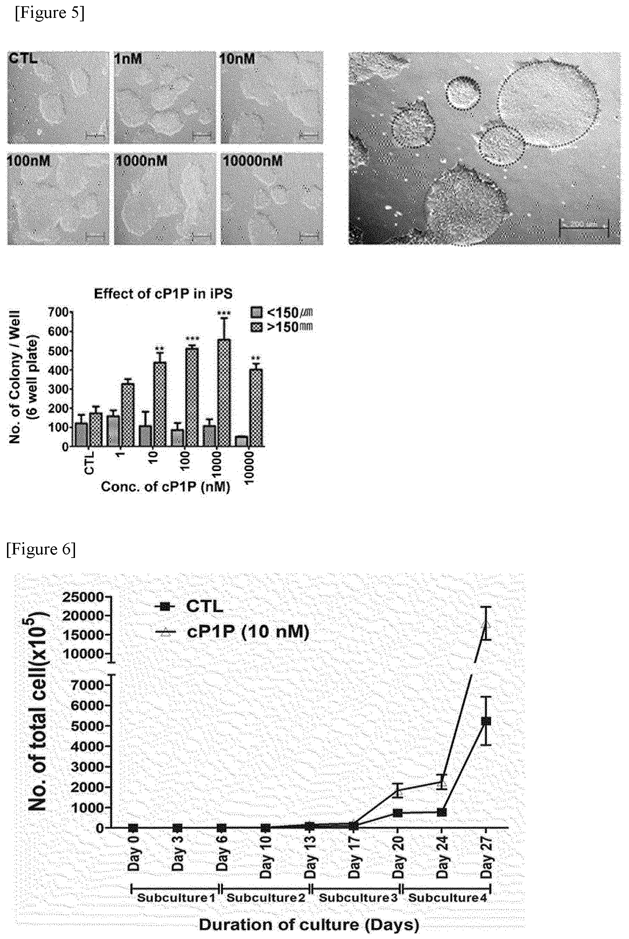

As a result, as shown in FIG. 5, when hPSCs were treated with 10 nM or more of cP1P, the number of colonies with a size of 150 mm or more was significantly increased (P < 0.01) compared to the control group.

### Example 5. Confirmation of whether hPSCs proliferation ability change by cP1P treatment is maintained during subculture

In order to check whether the change in hPSCs proliferation ability by cP1P treatment is maintained during subculture, hPSCs were continuously treated with 10 nM cP1P in the same manner as in Example 1, and after subculture 3 times, the proliferation change of hPSCs was confirmed.

As a result, as shown in FIG. 6, it was confirmed that when hPSCs were treated with cP1P, the total number of cells increased significantly as a subculture was performed (P < 0.01).

### Example 6. Confirmation of change in hPSC cell cycle by cP1P treatment

In order to confirm a change in hPSC cell cycle by cP1P treatment, flow cytometer, apoptosis analysis, and expression analysis of cell cycle regulatory genes were performed.

For flow cytometry, hPSCs were treated with 10 nM cP1P in the same manner as in Example 1, and hPSCs were separated using cell dissociation buffer (Thermo Fisher Scientific US, A1110501), and harvested cells were washed three times with DPBS (pH 7.4). After washing, the supernatant was removed. During washing, cold 70% (v/v) ethanol was added dropwise to the cell pellet. The cells were fixed at 4°C for 1 hour and washed 3 times with DPBS (pH 7.4). After centrifugation at 2,000 rpm for 5 minutes, 50 uL of 100 µg/ml RNase (Sigma-Aldrich US, R4642) was added. They were mixed well and added with 200 µl of PI (propidium iodide) (Sigma-Aldrich US, P4170) (50 µg/ml) to stain the DNA. Then, the DNA content was measured using flow cytometry (BD).

In order to confirm apoptosis, hPSCs were treated with 10 nM cP1P in the same manner as in Example 1, hPSCs were separated using cell dissociation buffer (Thermo Fisher Scientific US, A1110501), and harvested cells were washed with DPBS (pH 7.4) 2 times. After centrifuged at 2,000 rpm for 5 minutes. The cells were resuspended at 1 × 10⁵ cells per 100 ul of 1× binding buffer to which 10× binding buffer (0.1 M HEPES, 1.4 M NaCl, 25 mM CaCl₂, pH 7.4) was diluted. They were added with 5 ul each of Annexin V (BD bioscience US, 556422) and 7-AAD (BD bioscience US, 1559925) and mixed slowly. They were cultured for 15 minutes in the dark at room temperature and added with 400 ul of 1× binding buffer. Then, analysis was performed using the Annexin V apoptosis detection kit (BD Pharmingen US, 556547).

For expression analysis of cell cycle regulatory genes, hPSCs were treated with 10 nM cP1P in the same manner as in Example 1. After hPSCs were washed with PBS, hPSCs were fixed in 4% (w/v) PFA (Santa Cruz Biotechnology US, 30525-89-4) for 5 minutes, or after the protein of hPSC cells was recovered, followed by electrophoresis. The expression of pluripotency genes was analyzed (immunofluorescence analysis or Western blot analysis) using anti-REX1(Abcam UK, ab50828), anti-OCT4(Santa Cruz Biotechnology US, sc-5279), anti-SOX2(Abcam UK, ab97959), anti-NANOG(Cell signaling US, 4893s), anti-E-cadherin(Cell signaling US, 24E10), or anti-SSEA4(Merck millipore US, 90231) antibody. Meanwhile, the control group was tested in the same manner as above, except that hPSCs were treated with DMSO (AppliChem GE, A3672,0100).

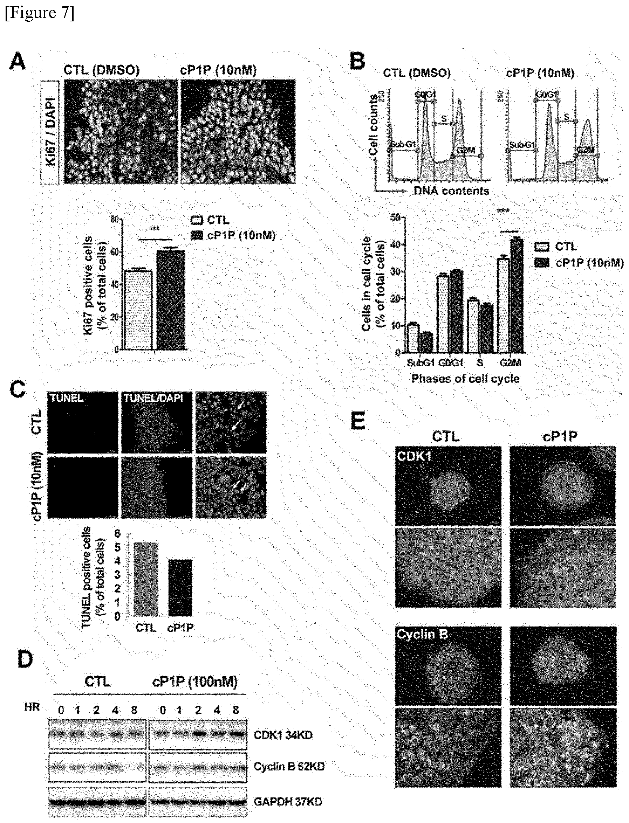

As a result, as shown in FIG. 7A, it was confirmed that when hPSCs were treated with 10 nM cP1P, cell proliferation increased by about 12% or more compared to the control group (control group (48%) and experimental group (60%)). As shown in FIG. 7B, when hPSCs were treated with 10 nM cP1P, the number of cells corresponding to the G2/M phase in which cell proliferation was actively occurring was significantly increased compared to the control group (P < 0.01), and when hPSCs were treated with 10 nM cP1P, the number of cells belonging to the sub-GO phase corresponding to apoptotic cells was significantly reduced compared to the control group (P > 0.05).

In addition, as shown in FIGS. 7C to 7E, when hPSCs were treated with 10 nM cP1P, the expression of genes (CDK1, Cyclin B) regulating the cell cycle was increased compared to the control group.

### Example 7. Apoptosis and cytotoxicity assay

In order to confirm whether apoptosis and cytotoxicity were induced when hPSCs were treated with cP1P, hPSCs were treated with 10 nM or 100 nM cP1P for 5 days in the same manner as in Example 6, and then Annexin V apoptosis detection kit (BD Pharmingen US, 556547) was used for analysis.

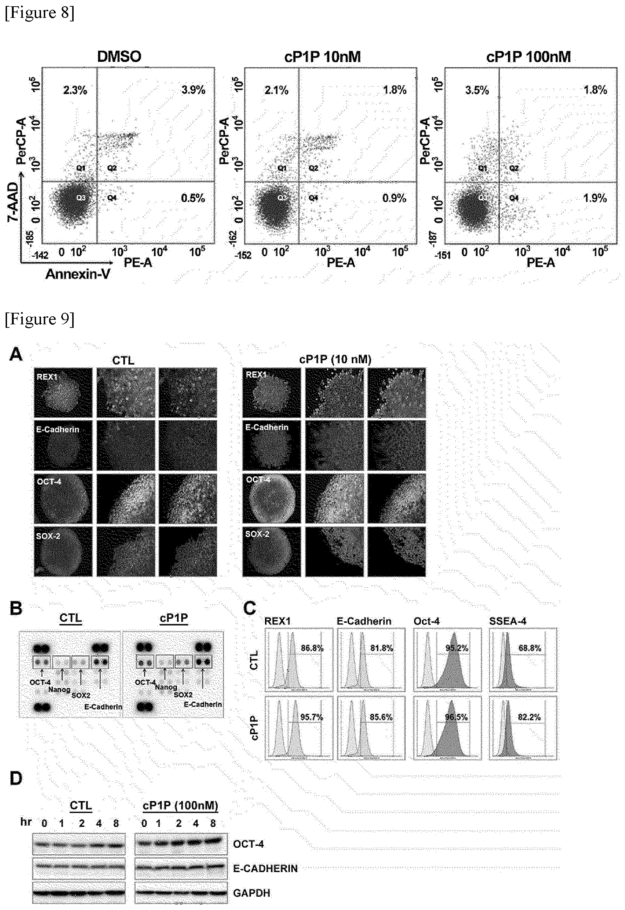

As a result, as shown in FIG. 8, it was confirmed that when hPSCs were treated with cP1P (cP1P 10 nM: 0.9%/3.9% and 100 nM: 1.9%/5.3%), apoptosis was reduced and the cytotoxicity was also significantly lowered compared to the control group (DMSO: 0.5%/6.2%) by Annexin V, an early apoptosis marker, and 7-AAD, a late apoptosis marker.

### Example 8. Confirmation of increase in the pluripotent properties of hPSCs by cP1P treatment

In order to confirm whether the pluripotent properties of hPSCs were increased by cP1P treatment, the following method was performed.

Specifically, hPSCs were treated with 100 nM cP1P in the same manner as in Example 1. Immunofluorescence analysis was performed in the same manner as in Example 6. Expression of pluripotency-related genes was analyzed using a pluripotent array kit (R&D System US, ARY101). Flow cytometry was performed in the same manner as in Example 6.

As a result, as shown in FIG. 9A, the expression of pluripotency marker genes REX1, E-Cadherin, OCT4 and SOX2 in the experimental group treated with cP1P was increased compared to the control group. As shown in FIG. 9B, the spots of pluripotency-related genes OCT4, NANOG, SOX2 and E-Cadherin in the cP1P-treated group were detected to be high compared to the control group. As shown in FIG. 9D, protein expression of pluripotency-related genes OCT4 and E-Cadherin in cP1P-treated group was increased compared to the control group. As shown in FIG. 9C, protein expression of pluripotency-related genes REX1 (86.8% -> 95.7%), E-Cadherin (81.8% - >85.6%), OCT4 (95.2% -> 96.5%) and SSEA-4 (68.8% -> 82.2%) in cP1P-treated group was increased compared to the control group.

### Example 9. Analysis of the effect of cP1P long-term treatment on hPSCs

Since long-term culturing of human PSCs may result in changes such as a decrease in the growth rate or microscopic changes in the properties of stem cells, it was investigated whether the characteristics of hPSCs were changed when treating with cP1P for a long period of time.

Specifically, hPSCs were treated and cultured with 10 nM cP1P or were treated and cultured with DMSO as a control group in the same manner as in Example 1. The passage in which cP1P was first treated was defined as 0 and cultured up to a total of 15 passages. After the cells were recovered, and RNAs were extracted. The RNA sequencing analysis was performed with a next-generation sequencing analyzer (Illumina HiSeq 2500) by DGIST (Daegu Gyeongbuk Institute of Science and Technology) asked to analyze the cells.

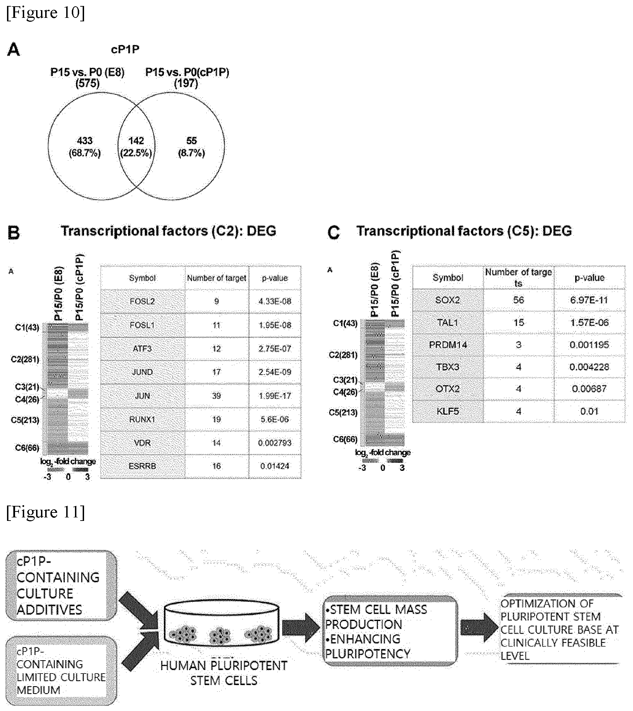

As a result, compared with the gene expression of the initial passage (passage 0), as shown in FIG. 10, 197 gene expression changes (DEG) were confirmed in cP1P-treated group, and 575 gene expression changes were confirmed in no cP1P-treated group.

The gene expression change group was divided into a group with increased expression (C2) and a group with decreased expression (C5) after 15 passage culture, and gene ontology thereof was analyzed (FIGS. 10B and 10C). In the control group not treated with cP1P, the gene expression of cluster 2 (C2), a gene group responding to the oxidative stress and apoptotic process, was increased, but when cP1P was treated for a long time, C2 gene expression was decreased. Therefore, it was confirmed that the long-term treatment of cP1P has the effect of inhibiting apoptosis and reducing the oxidative stress that increases during long-term culture. In addition, in the control group not treated with cP1P, the expression of the cluster 5 (C5) gene, which is a gene group related to cell development, embryo development, and cell proliferation, was decreased. Accordingly, it was confirmed that the developmental function was defective. However, in the case of long-term treatment of cP1P, the expression of the C5 gene was not decreased and was stably maintained, so it was considered to have an effect of maintaining the expression of a gene group capable of initiating a normal development process.

In other words, it was confirmed that treatment with cP1P during the long-term passage of human PSCs causes a significant effect of inhibiting apoptosis, reducing oxidative stress that increases during long-term culture, enhancing pluripotency and maintaining proliferation ability.

### [Mode for Invention]

In a specific embodiment of the present invention, it was confirmed that when human PSCs (hPSCs) were treated with cP1P, the cell proliferation ability was significantly increased compared to the case of treatment with phytosphingosine-1-phosphate (P1P) or sphingosine-1-phosphate (SIP).

In another example, it was confirmed that when hPSCs were treated with cP1P, the undifferentiation ability was well maintained compared to the control group.

In another example, it was confirmed that when hPSCs were treated with cP1P, the number of colonies was increased, and the colony size was increased compared to the control group.

In another example, it was confirmed that when hPSCs were treated with cP1P, the total number of cells was significantly increased as passages passed compared to the control group.

In another example, it was confirmed that when hPSCs were treated with cP1P, the expression of the genes (CDK1, Cyclin B) regulating the cell cycle was increased compared to the control group.

In another example, it was confirmed that when hPSCs were treated with cP1P, apoptosis was reduced, and cytotoxicity was also significantly lowered compared to the control group.

In another example, it was confirmed that when hPSCs were treated with cP1P, the pluripotent properties of cells were increased compared to the control group.

In another example, it was confirmed that when hPSCs were treated with cP1P for a long time, the pluripotent properties of cells were increased compared to the control group.

In another example, it was confirmed that when hPSCs were treated with cP1P for a long time, the pluripotent properties of cells were increased compared to the control group.

Hereinafter, the present invention is described in detail.

All technical terms used in the present invention, unless otherwise defined, have the meaning as commonly understood by one of ordinary skill in the art of the present invention.

One aspect of the present invention relates to a composition for promoting stem cell proliferation, the composition including o-cyclic phytosphingosine-1-phosphate (cP1P) or a pharmaceutically acceptable salt thereof as an active ingredient.

cP1P, sphingosine-1-phosphate (SIP) and phytosphingosine-1-phosphate (PIP) described herein are known substances, and their chemical structures are disclosed in FIG. 1.

The cP1P compound according to the present application, a pharmaceutically acceptable salt thereof, or a solvate thereof cannot be prepared using conventional knowledge known in the field of organic chemistry. For example, PIP may be prepared using a manner described in (S. Li, W.K. Wilson, G.J. Schroepfer, Chemical synthesis of D-ribo-phytosphingosine-1-phosphate, potential modulator of cellular processes. J. Lipid Res. 40: 117-125, 1999). However, it is different from the technology for synthesizing cP1P of the present application. The synthesis of cP1P of the present application is possible only by the method disclosed in Korea Patent Registration No. 10-1340556 (Novel phytospingosine-1-phosphate derivatives, a process for the preparation thereof, and a composition for hair tonic or treating or preventing hair loss comprising the same).

The salt is physiologically acceptable and does not cause a typical allergic reaction or a reaction similar thereto when administered to humans. The salt is preferably an acid addition salt formed by a free acid. The free acid may be an organic acid or an inorganic acid. The organic acids may include, but is not limited to, citric acid, acetic acid, lactic acid, tartaric acid, maleic acid, fumaric acid, formic acid, propionic acid, oxalic acid, trifluoroacetic acid, benzoic acid, gluconic acid, metasulfonic acid, glycolic acid, succinic acid, 4-toluenesulfonic acid, glutamic acid and aspartic acid. Further, the inorganic acid may include, but is not limited to, hydrochloric acid, hydrobromic acid, sulfuric acid and phosphoric acid. In one embodiment according to the present application, the pharmaceutically acceptable salt may exist as an acid addition salt in which the cP1P, SIP, or PIP compound forms a salt with a free acid. Further, the cP1P, SIP, or PIP compound according to the present application may include all salts, hydrates, and solvates that can be prepared by conventional methods as well as pharmaceutically acceptable salts.

As used herein, the term "stem cell" collectively refers to a cell with multiple differentiation ability in which one cell may be produced to different types of cells, and can regenerate cells in damaged areas of the human body. Stem cells have the selfrenewal ability to continuously generate the same cells as themselves, the differentiation ability to differentiate into functional specific cells in a specific environment, and the immune modulatory ability to regulate the immune response by reacting with immune cells. The types of stem cells can be divided into pluripotent stem cells, which have the ability to differentiate into about 200 types of cells that make up the human body depending on the area of the cell to be differentiated, and specialized tissue-specific stem cells to differentiate into specific types of cells. In addition, the types of stem cells can be divided into embryonic stem cells obtained from embryos or blastocytes starting from a fertilized egg and adult stem cells obtained from each tissue of a newborn or adult body after the development process has been completed.

The stem cells herein may be embryonic stem cells or induced pluripotent stem cells, and the stem cells may be obtained using any method commonly known in the art.

As used herein, the term "embryonic stem cell" refers to a cell in which an inner cell mass is extracted from a blastocyst embryo just before implantation of a fertilized egg in the mother's uterus and cultured *in vitro,* indicating a cell that can be pluripotent or totipotent capable of differentiating into cells of all tissues of the individual, and, in a broad sense, includes embryoid bodies derived from embryonic stem cells. The embryoid body is an intermediate structure formed by stem cells in the process of spontaneous differentiation of embryonic stem cells into various tissue types and is in the form of an aggregate formed during culturing of embryonic stem cells. Meanwhile, the embryonic stem cells of the present invention may be derived from mammals including humans and are preferably human embryonic stem cells.

Embryonic stem cells can differentiate into ectoderm, mesoderm and endoderm stem cells.

As used herein, the term "differentiation" refers to a phenomenon in which the structure or function of a cell is specialized while the cell divides and proliferates and grows. After pluripotent embryonic stem cells are differentiated into lineage-defined progenitor cells (e.g., ectoderm cells, mesoderm cells, or endoderm cells), the cells can be further differentiated into other types of progenitor cells (e.g., hemangioblasts), and then can be differentiated into terminally differentiated cells (e.g., vascular endothelial cells and vascular smooth muscle cells, etc.) that perform characteristic roles in specific tissues (e.g., blood vessels).

As used herein, the term "induced pluripotent stem cell" or "iPSC" refers to a cell having pluripotent differentiation obtained by treating somatic cells or already differentiated cells. Here, the treatment method includes, but is not limited to, a method of culturing under compound, genetic transformation or specific conditions. The term "human-induced pluripotent stem cell" or "hiPSC" refers to a cell having pluripotent differentiation by treating human somatic cells or human differentiated cells. The human-induced pluripotent stem cells may be derived from fibroblasts, but are not limited thereto, and may be derived from various sources such as blood. Further, the human-induced pluripotent stem cells may be produced by expressing reprogrammingrelated genes such as Oct4, Sox2, Klf4 and c-Myc in human fibroblasts. In this case, the expression of Oct4, Sox2, Klf4 and c-Myc genes may be derived through retroviral infection or an episomal system.

As used herein, cP1P, derivative compounds thereof, or salts thereof are used for promoting stem cell proliferation or growth. As a composition for promoting stem cell proliferation or a cell culture medium composition including the substance to achieve the effect disclosed herein, they may be used in addition to the usual medium used for stem cell culture.

When cP1P, derivative compounds thereof or salts thereof are added to the composition for promoting stem cell proliferation herein, the proliferation of stem cells may be promoted, stem apoptosis may be inhibited, the number and size of stem cell colonies may be increased, and the pluripotency of stem cells may be promoted, and preferably the proliferation of embryonic stem cells or induced pluripotent stem cells may be promoted, apoptosis may be inhibited, the number and size of colonies may be increased, and pluripotency (sternness, naive state) may be enhanced.

The concentration range of cP1P included in the composition for promoting stem cell proliferation may be 0.1 nM to 10,000 nM, preferably 0.5 to 200 nM, and cP1P in the concentration range may be added to the stem cell culture medium.

The cP1P compound, a pharmaceutically acceptable salt thereof, or a solvate thereof according to the above may be included in an appropriate concentration depending on the type of specific cell of interest, as long as it meets the purpose of the present application.

When the stem cells are cultured in a cell culture vessel added with the composition including the cP1P as an active ingredient, the stem cells in the cell culture vessel may effectively form and grow a plurality of colonies from single cells, and preferably embryonic stem cells, or induced pluripotent stem cells may be grown by forming colonies.

When stem cells form colonies and grow, they are similar to the form in which stem cells grow *in vivo.* Thus, stem cells are cultured using a composition including cP1P as an active ingredient to obtain stem cells having characteristics similar to those grown *in vivo.*

The medium used for culturing the stem cells herein may be used without limitation as long as it is a medium well known to those skilled in the art. The medium may be artificially synthesized and prepared, or a commercially prepared medium may be used. Examples of commercially prepared media include DMEM (Dulbecco's Modified Eagle's Medium), MEM (Minimal Essential Medium), BME (Basal Medium Eagle), RPMI 1640, F-10, F-12, α-MEM (α-Minimal essential Medium), G-MEM (Glasgow's Minimal Essential Medium), IMDM (Isocove's Modified Dulbecco's Medium), or MEF, but is not limited thereto.

The cP1P compound, a pharmaceutically acceptable salt thereof, or a solvate thereof according to the above may be included in an appropriate concentration depending on the type of specific cell of interest, as long as it meets the purpose of the present application.

Meanwhile, when the cP1P compound, a pharmaceutically acceptable salt thereof, or a solvate thereof according to the present application is added to the medium (or culture medium), the proliferation of stem cells was achieved in spite of a serum-free medium without serum components or a low-serum medium with reduced serum components

The composition for promoting stem cell proliferation of the present application may be one containing 0.1 to 3% by weight of serum-free or serum components.

The serum-free medium means any culture medium that does not contain more than a certain amount of serum (animal-derived serum) derived from animals including humans. For example, the serum-free medium may contain less than 0.1% by weight or less than 0.01% by weight of animal-derived serum based on the total composition content, and specifically may not contain animal-derived serum.

The present application provides a composition for adding a stem cell medium or a serum-free medium composition including a cP1P compound, a pharmaceutically acceptable salt thereof, or a solvate thereof instead of animal-derived serum required for the proliferation and culture of stem cells. Thus, the present invention may stably proliferate and culture stem cells to the extent that animal-derived serum can be replaced, and it is possible to establish a reproducible test and production process.

In the low serum medium, 0.1 to 3% by weight of fetal bovine serum (FBS) is added to the serum commonly used for cell culture, and a compound having a component similar to that of animal-derived serum, for example, bovine pituitary extract (BPE) and the like may also be used.

As used herein, the term "stem cell" is as described above, and is preferably derived from various adult tissues and bone marrow-derived cells such as bone marrow, adipose tissue, cord blood, peripheral blood, neonatal tissues, placenta, etc. but is not limited thereto.

In another aspect, the present invention relates to a composition for addition to a stem cell culture medium, the composition including cP1P or a pharmaceutically acceptable salt thereof as an active ingredient.

The description of the composition for promoting stem cell proliferation can be equally applied to the composition for addition to a stem cell culture medium as long as it does not deviate from the essence of the composition for addition to a stem cell culture medium.

The stem cells may be embryonic stem cells or induced pluripotent stem cells.

The concentration range of cP1P included in the composition for addition to the stem cell culture medium may be 0.1 nM to 10,000 nM, preferably 0.5 to 200 nM, and cP1P in the concentration range may be added to the stem cell culture medium.

In another aspect, the present invention relates to a method for culturing a stem cell, the method including a step of culturing stem cells by treating the composition for promoting stem cell proliferation to stem cells.

The description of the composition for promoting stem cell proliferation can be equally applied to the composition for addition to a stem cell culture medium as long as it does not deviate from the essence of the method for culturing stem cells.

The stem cells may be embryonic stem cells or induced pluripotent stem cells.

When the composition for promoting stem cell proliferation is treated in stem cells followed by culturing, the proliferation of stem cells may be promoted, stem apoptosis may be inhibited, the number and size of stem cell colonies may be increased, and the pluripotency of stem cells may be promoted, and preferably the proliferation of embryonic stem cells or induced pluripotent stem cells may be promoted, apoptosis may be inhibited, the number and size of colonies may be increased, and pluripotency (sternness, naive state) may be enhanced.

In addition, when the composition for promoting stem cell proliferation is treated in stem cells followed by culturing, stem cells may form colonies and grow in a cell culture vessel, preferably embryonic stem cells, or induced pluripotent stem cells may form colonies and grow.

In another aspect, the present invention relates to a kit for inhibiting stem apoptosis in an *in vitro* environment, the kit including the composition for promoting stem cell proliferation.

The description of the composition for promoting stem cell proliferation can be equally applied to the kit for inhibiting stem apoptosis in an *in vitro* environment as long as it does not deviate from the essence of the kit for inhibiting stem apoptosis in an *in vitro* culture environment.

The active ingredient included in the kit according to the present application may refer to the above-mentioned description and may include additional ingredients and usage for the desired effect in an *in vitro* culture environment.

The stem cells may be embryonic stem cells or induced pluripotent stem cells.

The concentration of cP1P included in the kit for inhibiting stem apoptosis in the *in vitro* culture environment may be 0.1 nM to 10,000 nM, but may preferably be 0.5 to 200 nM, and cP1P in the concentration may be added to the kit for inhibiting stem apoptosis in the *in vitro* culture environment but is not limited thereto.

When stem cells are cultured in the kit for inhibiting stem apoptosis in the *in vitro* culture environment including the composition for promoting stem cell proliferation, stem apoptosis may be suppressed and inhibited, the number and size of stem cell colonies may be increased, and the pluripotency of stem cells (sternness, naive state) may be enhanced.

In another aspect, the present invention relates to a kit for promoting stem cell proliferation in an *in vitro* environment, the kit including the composition for promoting stem cell proliferation.

The description of the composition for promoting stem cell proliferation can be equally applied to the kit for promoting stem cell proliferation in an *in vitro* culture environment as long as it does not deviate from the essence of the kit for promoting stem cell proliferation in an *in vitro* culture environment.

The active ingredient included in the kit according to the present application may refer to the above-mentioned description and may include additional ingredients and usage for the desired effect in an *in vitro* culture environment.

The stem cells may be embryonic stem cells or induced pluripotent stem cells.

The concentration of cP1P included in the kit for promoting stem cell proliferation in the *in vitro* culture environment may be 0.1 nM to 10,000 nM, but may preferably be 0.5 to 200 nM, and cP1P in the concentration may be added to the kit for inhibiting stem apoptosis in the *in vitro* culture environment but is not limited thereto.

When stem cells are cultured in the kit for promoting stem cell proliferation in the *in vitro* culture environment including the composition for promoting stem cell proliferation, the proliferation of stem cells may be promoted, and pluripotency may be enhanced.

In another aspect, the present invention relates to a method for promoting stem cell proliferation, the method including a step of culturing stem cells by adding o-cyclic phytosphingosine-1-phosphate (cP1P) or a pharmaceutically acceptable salt thereof to a stem cell culture medium.

The description of the composition for promoting stem cell proliferation can be equally applied to the method for promoting stem cell proliferation as long as it does not deviate from the essence of the method for promoting stem cell proliferation.

The stem cells may be embryonic stem cells or induced pluripotent stem cells.

When culturing stem cells by adding the cP1P or a pharmaceutically acceptable salt thereof to a stem cell culture medium, stem apoptosis may be inhibited, the number and size of stem cell colonies may be increased, and the pluripotency of stem cells may be promoted, and preferably the proliferation of embryonic stem cells or induced pluripotent stem cells may be promoted, apoptosis may be inhibited, the number and size of colonies may be increased, and pluripotency (sternness, naive state) may be enhanced. Accordingly, the proliferation of stem cells may be significantly promoted.

### [Industrial Applicability]

The present invention relates to a pluripotent composition for promoting stem cell proliferation and a composition for addition to a stem cell culture medium, the composition including cP1P or a pharmaceutically acceptable salt thereof as an active ingredient, and the composition for promoting stem cell proliferation and the composition for addition to a stem cell culture medium are used for culturing stem cells to enhance sternness (sternness), promote proliferation, and inhibit apoptosis so that it is useful in the pharmaceutical industry.

## Claims

1. A composition for promoting stem cell proliferation, the composition comprising o-cyclic phytosphingosine-1-phosphate (cP1P) or a pharmaceutically acceptable salt thereof as an active ingredient

2. The composition for promoting stem cell proliferation of claim 1, wherein the cP1P has a concentration of 0.1 nM to 10,000 nM.

3. The composition for promoting stem cell proliferation of claim 1, wherein the stem cells are embryonic stem cells or induced pluripotent stem cells.

4. The composition for promoting stem cell proliferation of claim 1, wherein the composition promotes the proliferation of stem cells and inhibits apoptosis.

5. The composition for promoting stem cell proliferation of claim 1, wherein the composition increases the number of stem cell colonies and a size of the colonies.

6. The composition for promoting stem cell proliferation of claim 1, wherein the composition strengthens pluripotency of stem cells (sternness, naive state).

7. The composition for promoting stem cell proliferation of claim 1, wherein the composition for culturing stem cells contains 0.1 to 3% by weight of serum-free or serum components.

8. A composition for addition to a stem cell culture medium, the composition comprising cP1P or a pharmaceutically acceptable salt thereof as an active ingredient.

9. The composition for addition to a stem cell culture medium of claim 8, wherein the cP1P has a concentration of 0.1 nM to 10,000 nM.

10. The composition for addition to a stem cell culture medium of claim 8, wherein the stem cells are embryonic stem cells or induced pluripotent stem cells.

11. A method for stem cell culture comprising a step of culturing stem cells by treating the composition of claim 1 to the stem cells.

12. The method for stem cell culture of claim 11, wherein the stem cells are embryonic stem cells or induced pluripotent stem cells.

13. A kit for inhibiting stem apoptosis in an *in vitro* culture environment, the kit comprising the composition of claim 1.

14. The kit for inhibiting stem apoptosis in an *in vitro* culture environment of claim 13, wherein the stem cells are embryonic stem cells or induced pluripotent stem cells.

15. A kit for promoting stem cell proliferation in an *in vitro* culture environment, the kit comprising the composition of claim 1.

16. A method for promoting stem cell proliferation, the method comprising a step of culturing stem cells by adding o-cyclic phytosphingosine-1-phosphate (cP1P) or a pharmaceutically acceptable salt thereof to a stem cell culture medium.

17. The method for promoting stem cell proliferation of claim 16, wherein the cP1P has a concentration of 0.1 nM to 10,000 nM.

18. The method for promoting stem cell proliferation of claim 16, wherein the stem cells are embryonic stem cells or induced pluripotent stem cells.
